Europäisches Patentamt

European Patent Office

Office européen des brevets

(11) Veröffentlichungsnummer : **0 035 704**
**B1**

# (12) EUROPÄISCHE PATENTSCHRIFT

(45) Veröffentlichungstag der Patentschrift :
26.01.83

(21) Anmeldenummer : 81101398.6

(22) Anmeldetag : 26.02.81

(51) Int. Cl.³ : **C 07 D251/28**

(54) Verfahren zur Herstellung von 2,4,6-Trifluortriazin-(1,3,5).

(30) Priorität : 08.03.80 DE 3008923

(43) Veröffentlichungstag der Anmeldung :
16.09.81 Patentblatt 81/37

(45) Bekanntmachung des Hinweises auf die Patenterteilung : 26.01.83 Patentblatt 83/04

(84) Benannte Vertragsstaaten :
CH DE FR GB IT LI

(56) Entgegenhaltungen :
DE A 2 643 251
DE A 2 643 335
DE A 2 729 762
DE A 2 814 450

(73) Patentinhaber : **BAYER AG**
**Zentralbereich Patente, Marken und Lizenzen**
**D-5090 Leverkusen 1, Bayerwerk (DE)**

(72) Erfinder : **Klauke, Erich, Dr.**
**Eichendorffweg 8**
**D-5062 Odenthal (DE)**
Erfinder : **Kysela, Ernst, Dr.**
**Virchowstrasse 14**
**D-5060 Berg.-Gladbach 3 (DE)**
Erfinder : **Stüwe, Arnd, Dr.**
**Pommernweg 11**
**D-2212 Brunsbuettel (DE)**
Erfinder : **Dorlars, Alfons, Dr.**
**Mozartstrasse 32**
**D-5090 Leverkusen (DE)**

EP 0 035 704 B1

# 0 035 704

## Verfahren zur Herstellung von 2,4,6-Trifluortriazin-(1,3,5)

Gegenstand der vorliegenden Erfindung ist ein Verfahren zur Herstellung von 2,4,6-Trifluortriazin-(1,3,5) (TFT) aus 2,4,6-Trichlortriazin-(1,3,5) (TCT) bzw. aus gemischtchlorierten-fluorierten 1,3,5-Triazinen durch Fluorierung mit Natriumfluorid in dipolaren aprotischen Lösungsmitteln.

Es ist bekannt, TFT aus TCT durch Fluorierung mit Natriumfluorid in dipolaren, aprotischen Lösungsmitteln herzustellen (C.W. Tullock and D.D. Coffman, J. Org. Chem. 25, 2016 (1960)) : zu einer Suspension von Natriumfluorid in Tetramethylensulfon wird TCT gegeben und die Reaktionsmischung stufenweise von 43 °C bis 248 °C erwärmt. Das entstehende TFT destilliert aus der Reaktionsmischung. Man erhält TFT in einer Ausbeute von 74 % der Theorie.

Für eine technische Nutzung besitzt dieses Verfahren erhebliche Nachteile :

Beim Erwärmen der Reaktionsmischung tritt beim Einsetzen der Reaktion ein kräftiger Temperaturanstieg auf, der bei Großansätzen nur schwer zu beherrschen ist.

Die hohe Reaktionstemperatur von zuletzt 248 °C erfordert kostspielige apparative Maßnahmen.

Es wurde nun gefunden, daß man diese Nachteile bei der Herstellung von TFT aus TCT bzw. aus gemischtchlorierten-fluorierten 1,3,5-Triazinen mit Natriumfluorid in einem dipolaren, aprotischen Lösungsmittel vermeiden kann und gleichzeitig zu vorteilhafteren Ergebnissen kommt, wenn man TCT oder gemischtchlorierte-fluorierte 1,3,5-Triazine oder Mischungen davon in eine Suspension von Natriumfluorid in einem dipolaren, aprotischen Lösungsmittel dosiert, die auf eine Temperatur zwischen 120 °C und 220 °C erwärmt ist und die gegebenenfalls ein weiteres Lösungsmittel enthält.

Bei der Umsetzung im dipolaren, aprotischen Lösungsmittel arbeitet man vorzugsweise zwischen 130 und 180 °C und insbesondere zwischen 140 und 160 °C.

TCT kann als Schmelze, Suspension oder Lösung zudosiert werden. Vorteilhaft ist es, das TCT gelöst oder suspendiert in einem der in der Reaktion eingesetzten Lösungsmittel, oder in einer Mischung dieser Lösungsmittel zuzudosieren. Ebenso vorteilhaft ist es, eine Lösung oder Suspension von TCT in gemischtchloriert-fluorierten 1,3,5-Triazinen zu verwenden.

Der Vorteil des Zudosierens von TCT bei Reaktionstemperatur liegt für ein technisches Verfahren z.B. darin, daß man den Temperaturstoß vermeiden kann, der nach dem Stand der Technik auftritt, wenn alle Reaktionspartner vorgelegt und gemeinsam auf Reaktionstemperatur erwärmt werden. Die Ausbeuten an TFT liegen im Vergleich zum Stand der Technik überraschenderweise um mindestens 20 % höher.

TCT kann als Schmelze, Suspension oder Lösung zugegeben werden. Vorteilhaft ist es, das TCT gelöst oder suspendiert in einen der in der Reaktion eingesetzten Lösungsmittel, oder in einer Mischung dieser Lösungsmittel zuzudosieren. Ebenso vorteilhaft ist es, eine Lösung oder Suspension von TCT in gemischtchloriert-fluorierten 1,3,5-Triazinen zu verwenden. Als für die Durchführung der Reaktion geeignetes dipolares aprotisches Lösungsmittel kommen vorzugsweise in Frage : Tetramethylensulfon.

Als Zusatz zum jeweils verwendeten dipolaren, aprotischen Lösungsmittel eignen sich solche Lösungsmittel, die unter den Reaktionsbedingungen inert sind und die in einem Bereich sieden, der zwischen dem Siedepunkt des TFT und dem des verwendeten dipolaren, aprotischen Lösungsmittel liegt (sogenannte « Zwischensieder »). Bevorzugt sind Lösungsmittel im Siedebereich von 120 °C bis 200 °C. Als besonders bevorzugte Lösungsmittel seien genannt : halogenierte Kohlenwasserstoffe wie z.B. Chlorbenzol oder 1,2-Dichlorbenzol, oder Alkylbenzole wie o-, m-, p-Xylol oder deren Gemische.

Der Zusatz dieser zweiten Lösungsmittel zum Reaktionsgemisch bringt verschiedene Vorteile. Man erreicht einen gesteigerten Austrag an Produkt und dadurch eine höhere Ausbeute (> 95 % d. Th.) ; man erhält ein Produkt hoher Reinheit (Gehalt 99,5 %) und man kann bei drastisch reduzierten Reaktionstemperaturen arbeiten. Günstigerweise arbeitet man zwischen 120 °C bis 200 °C, bei Verwendung von 1,2-Dichlorbenzol in Tetramethylensulfon, vorzugsweise zwischen 130 °C bis 160 °C. Bemerkenswert ist, daß die Steigerung der TFT-Ausbeute ohne zusätzliche Natriumfluorid-Gaben unter Beibehaltung des bereits in der Literatur beschriebenen Mol Natriumfluorid/Mol TCT-Verhältnisses von ~ 3,6-3,7 gelingt. Weiterhin wird die Aufarbeitung der Reaktionsrückstände, z.B. die destillative Rückgewinnung des dipolaren aprotischen Lösungsmittels, wesentlich vereinfacht, da man geringe Restmengen TFT mit dem Zwischensieder glatt und vollständig entfernen kann (der TFT-haltige Zwischensieder kann in Folgeansätzen wiederverwendet werden, wie auch das rückgewonnene dipolare, aprotische Lösungsmittel). Außerdem erhält man ökologisch unbedenkliche Metallsalze, deren Entsorgung problemlos ist.

Es ist als ausgesprochen überraschend zu bezeichnen, daß beim Zusatz des zweiten Lösungsmittels, welches als sog. Zwischensieder bekanntlich eine Schlepperfunktion besitzt, das Endprodukt 2,4,6-Trifluor-triazin in hoher Reinheit ohne nennenswerten Anteil der Nebenprodukte 2,4-Difluor-6-chlortriazin (1,3,5) und 2-Fluor-4,6-dichlortriazin (1,3,5) anfällt.

Das als Zwischensieder eingesetzte zweite Lösungsmittel kann in einer Menge von 0,05-0,6 Mol, bezogen auf eingesetztes, dipolares aprotisches Lösungsmittel verwendet werden, vorzugsweise jedoch wie z.B. bei 1,2-Dichlorbenzol/Tetramethylensulfon in Mengen von 0,2-0,4 Mol. Im allgemeinen wird TCT mit NaF im Molverhältnis 1 : 3 bis 1 : 5 vorzugsweise 1 : 3, 3 bis 1 : 4 umgesetzt.

TFT ist ein vielseitig verwendbares Zwischenprodukt, das sich z.B. zur Herstellung von Reaktivfarbstoffen und Herbiziden eignet. Das erfindungsgemäße Verfahren sei anhand folgender Beispiele erläutert.

# 0 035 704

## Beispiel 1

454 g (10,8 Mol) NaF und 520 g Tetramethylensulfon werden in einem Schaufelreaktor vorgelegt und unter Rühren auf 160 °C erwärmt. Dann dosiert man innerhalb einer Stunde eine 140 °C heiße Lösung von 555 g (3 Mol) TCT in 300 g Tetramethylensulfon zu. Nachdem ca. 30-50 % eingetragen sind, beginnt TFT abzudestillieren. Nach Ende der Zugabe steigert man die Temperatur innerhalb einer Stunde auf 190 °C und unter Beibehaltung dieser Temperatur evakuiert man allmählich auf ca. 50 mbar. Man erhält 360 g TFT (89 % d. Th.).

## Beispiel 2

In einem 3 l Schaufelreaktor werden 900 g NaF (21,5 Mol) in 150 g Tetramethylensulfon (getrocknet durch Destillation) und 300 g 1,2-Dichlorbenzol vorgelegt. Bei einer Innentemperatur von ca. 140-150 °C wird in 60-75 Minuten eine Suspension von 1 107 g TCT (6 Mol) in 924 g Tetramethylensulfon (Suspensionstemperatur 30-35 °C) in den Schaufelreaktor dosiert.

Nach einer dosierten Suspensionsmenge von 800-820 g beginnt bei Normaldruck über eine Kolonne TFT abzudestillieren. Bis zur Beendigung der Suspensionsdosierung sind 350-400 g TFT abdestilliert.

Anschließend wird die Temperatur in 40-60 Minuten auf 180-190 °C erhöht. Unter diesen Bedingungen werden 693 g TFT ($\hat{=}$ 85,2 % d. Th. bezogen auf eingesetztes TCT) erhalten.

Es wird dann der Druck im Reaktor auf 450 mbar verringert, wobei noch 79 g TFT ($\hat{=}$ 10 % d. Th.) abdestillieren. Durch weiteres Steigern des Vakuums auf 100 mbar werden 295 g 1,2-Dichlorbenzol als Zwischensieder abdestilliert.

Bei weiterer Verringerung des Druckes im Reaktor werden 1 000 g Tetramethylensulfon rückgewonnen.

Im Schaufelreaktor verbleibt ein heller, pulvriger, geruchsloser Salzrückstand, der sich leicht aus dem Schaufelreaktor austragen läßt.

Ausbeuten :
TFT : 96,5 % d. Th. bezogen auf umgesetztes TCT (Gehalt : 99,5 %ig)
1,2-Dichlorbenzol : 98,3 % bez. auf den Einsatz (Gehalt : 97,9 %)
Tetramethylensulfon : 93,1 % bez. auf den Einsatz (Gehalt : 99 %ig)

## Beispiel 3

Analog Beispiel 2, anstelle der TCT-Suspension wird jedoch eine Mischung aus 8,4 % TFT, 20,3 % Difluorchlortriazin, 33,9 % Dichlorfluortriazin und 36,1 % TCT zudosiert (das in Beispiel 2 für die Suspension benötigte Tetramethylensulfon wird im Schaufelreaktor vorgelegt). Man erhält 375-390 g TFT ($\hat{=}$ 92-96 % d. Th.).

Die gemäß Beispiel 3 als Reaktionskomponente eingesetzte Mischung aus TFT, Difluorchlortriazin, Dichlorfluortriazin und TCT kann gemäß Beispiel 1-3 der DE-OS 27.02 625 wie folgt gewonnen werden :

200 g Cyanurchlorid und 100 g Cyanurfluorid werden mit 3 g Aktivkohle 30 Minuten bei 180 °C zusammengegeben. Man erhält dann ein Gemisch bestehend aus 8,4 % TFT, 20,3 % Difluorchlortriazin, 33,9 % Dichlorfluortriazin und 36,1 % TCT.

**Ansprüche**

1. Verfahren zur Herstellung von 2,4,6-Trifluortriazin-(1,3,5) (TFT) aus 2,4,6-Trichlortriazin-(1,3,5) (TCT) oder aus gemischtchlorierten-fluorierten 1,3,5-Triazinen durch Fluorierung mit NaF in einem dipolaren, aprotischen Lösungsmittel, dadurch gekennzeichnet, daß man TCT in eine auf 120 °C bis 220 °C erwärmte Suspension von NaF in einem dipolaren, aprotischen Lösungsmittel dosiert, welches gegebenenfalls ein weiteres Lösungsmittel enthält.

2. Verfahren nach Anspruch 1, dadurch gekennzeichnet, daß es sich bei dem dipolaren, aprotischen Lösungsmittel um Tetramethylensulfon handelt.

3. Verfahren nach Anspruch 1, dadurch gekennzeichnet, daß dem dipolaren, aprotischen Lösungsmittel ein weiteres Lösungsmittel zugesetzt ist, welches inert ist und dessen Siedepunkt zwischen den Siedepunkt des TFT und dem Siedepunkt des dipolaren, aprotischen Lösungsmittels liegt.

4. Verfahren nach Anspruch 1, dadurch gekennzeichnet, daß man TCT als Schmelze, oder gelöst oder suspendiert in einem der in der Reaktion eingesetzten Lösungsmittel oder einem Gemisch dieser Lösungsmittel einsetzt.

5. Verfahren nach Anspruch 1, dadurch gekennzeichnet, daß man TCT gemischt mit, oder gelöst in gemischtchloriert-fluorierten 1,3,5-Triazinen zudosiert, bzw. gemischtchlorierte-fluorierte 1,3,5-Triazine verwendet.

6. Verfahren nach Anspruch 1, dadurch gekennzeichnet, daß man als weitere Lösungsmittel solche

**0 035 704**

verwendet, deren Siedepunkte bevorzugt zwischen 120-200 °C liegen und die halogenierte Kohlenwasserstoffe oder Alkylbenzole sind.

7. Verfahren nach Anspruch 6, dadurch gekennzeichnet, daß der als weiteres Lösungsmittel eingesetzte halogenierte Kohlenwasserstoff Chlorbenzol oder 1,2-Dichlorbenzol ist und die Alkylbenzole o-, m-, p-Xylol oder deren Gemische sind.

8. Verfahren nach Anspruch 3, dadurch gekennzeichnet, daß man das zweite Lösungsmittel in einer Menge von 0,05-0,6 Mol bezogen auf das eingesetzte, dipolare, aprotische Lösungsmittel einsetzt.

## Claims

1. Process for the preparation of 2,4,6-trifluoro-1,3,5-triazine (TFT) from 2,4,6-trichloro-1,3,5-triazine (TCT) or from mixed chlorinated/fluorinated 1,3,5-triazines by fluorination with NaF in a dipolar, aprotic solvent, characterised in that TCT is metered into a suspension, warmed to 120 °C to 220 °C, of NaF in a dipolar, aprotic solvent, which optionally contains a further solvent.

2. Process according to Claim 1, characterised in that the dipolar, aprotic solvent is tetramethylenesulphone.

3. Process according to Claim 1, characterised in that a further solvent which is inert and which has a boiling point between the boiling point of TFT and the boiling point of the dipolar, aprotic solvent is added to the dipolar, aprotic solvent.

4. Process according to Claim 1, characterised in that TCT is employed as a melt or as a solution or suspension in one of the solvents employed in the reaction or in a mixture of these solvents.

5. Process according to Claim 1, characterised in that TCT is metered in as a mixture with or solution in mixed chlorinated/fluorinated 1,3,5-triazines, or mixed chlorinated/fluorinated 1,3,5-triazines are used.

6. Process according to Claim 1, characterised in that the further solvents used are preferably those which have boiling points between 120-200 °C and which are halogenated hydrocarbons or alkylbenzenes.

7. Process according to Claim 6, characterised in that the halogenated hydrocarbon employed as the further solvent is chlorobenzene or 1,2-dichlorobenzene and the alkylbenzenes are o-, m- or p-xylene, or mixtures thereof.

8. Process according to Claim 3, characterised in that the second solvent is employed in an amount of 0.05-0.6 mol, relative to the dipolar, aprotic solvent employed.

## Revendications

1. Procédé de production de 2,4,6-trifluorotriazine-(1,3,5) (TFT) à partir de 2,4,6-trichlorotriazine-(1,3,5) (TCT) ou de 1,3,5-triazines chloro-fluorées mixtes, par fluoration avec NaF dans un solvant aprotique dipolaire, caractérisé en ce qu'on verse de la TCT dans une suspension, chauffée à 120-220 °C, de NaF dans un solvant aprotique dipolaire qui contient éventuellement un autre solvant.

2. Procédé suivant la revendication 1, caractérisé en ce que le solvant aprotique dipolaire est la tétraméthylènesulfone.

3. Procédé suivant la revendication 1, caractérisé en ce qu'on ajoute au solvant aprotique dipolaire un autre solvant qui est inerte et dont le point d'ébullition se situe entre le point d'ébullition de la TFT et le point d'ébullition du solvant aprotique dipolaire.

4. Procédé suivant la revendication 1, caractérisé en ce qu'on utilise de la TCT à l'état fondu, ou bien dissoute ou en suspension dans l'un des solvants utilisés dans la réaction ou dans un mélange de ces solvants.

5. Procédé suivant la revendication 1, caractérisé en ce qu'on ajoute la TCT en mélange avec, ou en solution dans, des 1,3,5-triazines chloro-fluorées mixtes ou on utilise des 1,3,5-triazines chloro-fluorées mixtes.

6. Procédé suivant la revendication 1, caractérisé en ce qu'on utilise comme autres solvants des solvants dont les points d'ébullition se situent de préférence entre 120 et 200 °C et qui sont des hydrocarbures halogénés ou des alkylbenzènes.

7. Procédé suivant la revendication 6, caractérisé en ce que l'hydrocarbure halogéné utilisé comme autre solvant est le chlorobenzène ou le 1,2-dichlorobenzène et les alkylbenzènes sont le o-, m-, le p-xylène ou leurs mélanges.

8. Procédé suivant la revendication 3, caractérisé en ce qu'on utilise le second solvant en une quantité de 0,05 à 0,6 mole par rapport au solvant aprotique dipolaire utilisé.

4